# NEW EUROPEAN PATENT SPECIFICATION

(11) **EP 0 932 397 B2**
(45) Date of publication and mention of the opposition decision: **02.04.2014**
(45) Mention of the grant of the patent: 15.03.2006
(21) Application number: 97943453.7
(22) Date of filing: 23.09.1997
(51) Int. Cl.: A61K 9/72, A61K 9/10

(54) **MEDICINAL AEROSOL FORMULATIONS COMPRISING BUDESONIDE**
MEDIZINISCHE AEROSOLFORMULIERUNGEN ENTHALTEND BUDESONIDE
FORMULATIONS POUR AEROSOLD MEDICINAUX CONTENANT DU BUDESONIDE

(30) Priority: 27.09.1996 GB 9620187
(43) Date of publication of application: 04.08.1999
(73) Proprietor: MINNESOTA MINING AND MANUFACTURING COMPANY, St. Paul, MN 55133-3427 (US)
(72) Inventor: GOVIND, Nayna, Saint Paul, MN 55133-3427 (US); JINKS, Philip, A., Saint Paul, MN 55133-3427 (US); ROSS, Danna, L., Saint Paul, MN 55133-3427 (US); WARD, Gary, H., Saint Paul, MN 55133-3427 (US)
(74) Representative: Knowles, James Atherton
(86) International application number: PCT/US1997/016869
(87) International publication number: WO 1998/013031

(56) References cited:
- EP-A- 0 504 112
- EP-A- 0 533 731
- EP-A- 0 633 019
- EP-A2- 0 372 777
- EP-A2- 0 504 112
- WO-A-91/11495
- WO-A-94/21229
- WO-A-95/08603
- WO-A-95/27476
- WO-A-96/19198
- WO-A1-91/11495
- WO-A1-93/11747
- WO-A1-93/18746
- WO-A1-96/18384
- M.J. KONTNY ET AL.: 'ISSUES SURROUNDING MDI FORMULATION DEVELOPMENT WITH NON-CFC PROPELLANTS' JOURNAL OF AEROSOL MEDICINE vol. 4, no. 3, 1991, pages 181 - 187
- PAUL A. SANDERS, PH.D.: 'HANDBOOK OF AEROSOL TECHNOLOGY', vol. 2, 1979, LITTON EDUCATIONAL PUBLISHING, INC., USA, ISBN 0-442-27348-7 deel PAUL A. SANDERS: 'SOLVENCY', pages 165 - 187

## Description

This invention relates to medicinal aerosol formulations and in particular to aerosol formulations containing budesonide which are suitable for administration to the respiratory system of a patient.

Pharmaceutical suspension aerosol formulations are known which use a mixture of liquid chlorofluorocarbons as the propellant. Fluorotrichloromethane, dichlorodifluoromethane and dichlorotetrafluoroethane are the most commonly used propellants in aerosol formulations for administration by inhalation.

Chlorofluorocarbons (CFCs), however, have been implicated in the destruction of the ozone layer and their production is being phased out. Hydrofluoroalkanes, such as hydrofluoroalkane 134a (HFA 134a, 1,1,1,2-tetrafluoroethane) and hydrofluoroalkane 227 (HFA 227, 1,1,1,2,3,3,3-heptafluoropropane), are viewed as being more ozone friendly than many chlorofluorocarbon propellants; furthermore, they have low toxicity and vapor pressures suitable for use in aerosols.

W091/04011, W091/11495, W091/11496, W093/11745, W093/11747, W094/21228, W095/15151, W096/9816, W096/9831, EP-A-0372777, EP-A-0384371, EP-A-0518600, EP-A-0518601, EP-A-0550031, EP-A-O587790 and US-A-5492688 disclose aerosol formulations in which the propellant comprises a hydrofluoroalkane.

EP-A-0605578 discloses pharmaceutical aerosol compositions comprising a liquefied hydrofluoralkane, a powdered medicament and a polymer, soluble in the hydrofluoroalkane, having recurring structural units selected from amide containing units and carboxylic acid ester containing units. One formulation consists of HFA 227, budesonide and a polyvinylpyrrolidone/vinyl acetate copolymer and a further formulation additionally comprises polyethylene glycol.

W093/18746 discloses a pharmaceutical aerosol formulation consisting of HFA 227, budesonide, 1% by weight polyoxyethylene-25-glyceryl-trioleate and 1% by weight ethanol.

EP-A-0504112 discloses inter alia a formulation comprising 0.312% budesonide, 0.039% Myvacet 9-45, 1.171% Tween 60,11.50% ethanol and 86.978% HFA 227. The ethanol content of this formulation is sufficient to dissolve a substantial proportion of the budesonide and is likely to exhibit crystal growth of budesonide particles.

W094/21229 discloses formulations comprising 0.03% particulate budesonide, 0.05% dispersing aid and 99.92% propellant which was either HFA 134a or HFA 227. The dispersing aids are derived from acetyl-oligo-L-lactic acids. The ingredients were homogenized using ultrasound. After storage, each formulation was shaken by hand then observed on standing. Each of the suspensions is said to flocculate within 5 seconds after shaking ceased.

WO96/19198 discloses pharmaceutical aerosol formulations comprising a HFA propellant; a physiologically effective amount of a medicament for inhalation; and a surfactant which is a C₈-C₁₆ fatty acid or salt thereof, a bile salt, a phospholipid, or an alkyl saccharide.

Examples 2 and 11 disclose formulations comprising micronised budesonide, micronised sodium taurocholate and hydrofluoroalkane 134a. Example 11 additionally comprises ethanol.

EP-A-633019 discloses aerosol compositions using polyoxyethylene glyceryl fatty acid esters as suspension stabilisers and valve lubricants. The composition may contain hydrofluoroalkane 134a or 227, ethanol and drug, such as budesonide.

EP-A-0534731 discloses a pressurised aerosol composition comprising a liquefied hydrofluoroalkane, a powdered medicament dispersable therein and a polymer soluble in the liquefied hydrofluoroalkane, wherein the polymer includes recurring structural units, the units being selected from amide containing units and carboxylic acid ester containing units.

WO91/11495 discloses propellant gas mixtures suitable for use in medicinal preparations comprising two or more components, at least one of which is a partly fluorinated lower alkane.

Suspension formulations of budesonide have a propensity to rapidly form coarse flocs upon dispersion and redispersion which may deleteriously affect dosage reproducibility. There is also a tendency for budesonide to deposit from suspension onto the walls of the container.

The teaching of the state of the art does not provide a ready solution to these problems.

According to one aspect of the present invention there is provided a pharmaceutical aerosol formulation in suspension form suitable for administration to a patient by oral or nasal inhalation consisting of:
particulate budesonide,
hydrofluoroalkane propellant selected from HFA 134a, HFA 227 and mixtures thereof,
from about 1 to 2% by weight of an adjuvant having a Kauri-butanol value of at least 10 and
optionally from 0.0001 to 1% by weight of the formulation of one or more surfactants selected from oleic acid, sorbitan oleates and lecithin.

It has been found that it is possible to achieve stable suspensions of particulate budesonide by employing up to 2% of an adjuvant having a Kauri butanol value greater than 10 e.g. ethanol. In such formulations, the level of adjuvant is selected to decrease the propensity for rapid formation of coarse flocs and for deposition of drug on manufacturing equipment and on the internal surfaces of the container closure system of the inhaler. However, the levels are not so high as to cause significant solubilization of drug, leading to problems
of chemical degradation and particle size increase on storage.

It has been found that it is possible to achieve stable suspensions of particulate budesonide by employing a mixture ofHFA propellants by matching the density of the propellant mixture to be substantially identical to the density of budesonide. Such formulations are referred to herein as "density matched". The particles preferably have an average size in the range 1 to 10µm.

In addition to its use for the control of asthma, budesonide is particularly suited for nasal delivery in the treatment of allergic rhinitis. Formulations for this application preferably do not contain high levels of ethanol in order to avoid irritation of the nasal mucosa. Levels of about 1% by weight ethanol have been found not to produce irritation.

Formulations of the invention exhibit substantially no growth in particle size or change in crystal morphology of the drug over a prolonged period, are substantially and readily redispersible, and upon redispersion do not flocculate so quickly as to prevent reproducible dosing of the drug.

It has been found that budesonide particles will sink when suspended in 100% HFA 134a but float when suspended in 100% HFA 227.

It has been found that it is possible to match the density of budesonide using a propellant mixture of HFA's, particularly a mixture of HFA 134a and HFA 227. Suitable propellant mixtures comprise from 15 to 35%, HFA 227 and correspondingly 65 to 85% by weight HFA 134a.

Altough density matched mixtures of HFA propellants provide improved formulations of suspended budesonide compared with the use of single propellants (such mixtures do not necessarily prevent the formation of large flocs or prevent drug deposition on the walls of the container or equipment used in preparing the formulation. It has been found that the presence of ethanol may improve the properties of both density matched and other formulations of suspended budesonide. Ethanol may be present in a proportion which does not lead to crystal growth or produce irritation when inhaled, particularly when inhaled intranasally.

In addition, small amounts of surfactant, generally 0.0001 to 1%, preferably from 0.0005 to 0.01% by weight may provide improved properties, e.g., preventing particles adhering to surfaces and providing lubrication for valve components in contact with the formulation. The surfactant is selected from oleic acid, lecithin and sorbitan oleates, e.g., sorbitan monooleate, sorbitan sesquioleate and sorbitan trioleate. The preferred surfactant is oleic acid.

Ethanol is generally present in an amount of from 1 to 2% by weight. The budesonide is generally present to provide a dose of from 1 to 8mg/ml of formulation. Exemplary doses are 1,2,4 and 8 mg/ml. Such doses are achieved using a concentration of budesonide of from about 0.075 to 0.66% by weight of the formulation depending upon the precise formulation.

A preferred formulation in accordance with the invention consists of:
particulate budesonide
oleic acid
ethanol
HFA 134a
HFA 227

It is conventional practice when preparing aerosol formulations to mix the drug with the highest boiling point material and thereafter mix with the propellant. However, when making the formulations of the present invention it is important to ensure the budesonide does not come into contact with high concentrations, e.g., above 5% w/w, of ethanol since the drug would dissolve leading to instability and crystal growth problems in the final formulation. Preferably the maximum concentration of ethanol during formulation is less than 1%.

When preparing formulations of the invention with a content of up to 1% w/w ethanol, the concentration of ethanol at any stage in the presence of budesonide must be maintained no higher than this level. A procedure for preparing a budesonide suspension formulation by cold-filling is as follows:
a) Add all of the formulation quantity of HFA 134a and half of the formulation quantity ofHFA 227 to a batching vessel.
b) Prepare a first concentrate containing any surfactant and at least 85% of the formulation quantity of ethanol. Add this to the batching vessel.
c) Prepare a second concentrate containing the other half of the formulation quantity of HFA 227 and the remainder of the ethanol (i.e., no more than 1% w/w of the second concentrate), and add the micronized drug while mixing under high shear. Add the second concentrate to the batching vessel.

The invention will now be described with reference to the following Examples which employed micronized budesonide.

### Examples 1 to 3

Formulations were prepared using HFA 134a as propellant. Particulate budesonide was present at 0.33% by weight optionally with ethanol and surfactant as reported in the following table.

| Example | Surfactant | | |
|---|---|---|---|
| | % Ethanol | % Oleic Acid | % Span 85 |
| 1 | 1 | 0 | 0 |
| 2 | 1 | 0.01 | 0 |
| 3 | 1 | 0.05 | 0 |

All formulations gradually sedimented.

### Examples 4 to 11

The formulations reported in the following Tables were prepared in which the amounts are expressed in % w/w:

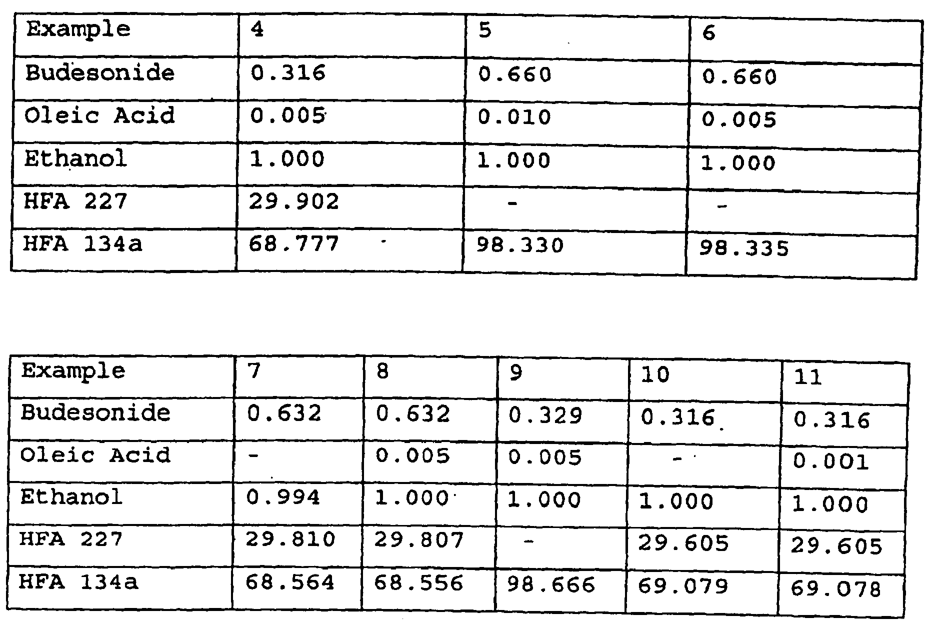

The budesonide particles in the formulations of 5, 6 and 9 sedimented.

Example 4 is a density matched stable formulation.

Examples 7, 8, 10 and 11 are density matched formulations. Example 11 exhibited less drug deposition than Example 10.

### Examples 12 to 16

The formulations reported in the following Table were prepared in which the amounts are expressed in % w/w.

| Example | 12 | 13 | 14 | 15 | 16 |
|---|---|---|---|---|---|
| Budesonide | 0.079 | 0.079 | 0.079 | 0.079 | 0.158 |
| Oleic Acid | - | 0.001 | 0.004 | 0.008 | 0.001 |
| Ethanol | 1.000 | 1.000 | 1.000 | 1.000 | 1.000 |
| HFA 227 | 29.676 | 29.676 | 29.675 | 29.674 | 29.652 |
| HFA 134a | 69.245 | 69.244 | 69.242 | 69.239 | 69.189 |

Examples 12 to 16 are density matched formulations. In the density matched formulations the floc matrix remains more evenly dispersed in the formulation than in the formulations containing HFA 134a and HFA 227 as the only propellant. Examples 13 and 14 exhibited less drug deposition than Examples 12 and 15.

### Examples 17 to 20

The formulations reported in the following Table were prepared in which the amounts are expressed in % w/w.

| Example | 17 | 18 | 19 | 20 |
|---|---|---|---|---|
| Budesonide | 0.328 | 0.323 | 0.321 | 0.318 |
| Oleic Acid | 0.001 | 0.001 | 0.001 | 0.001 |
| Ethanol | 1.000 | 1.000 | 1.000 | 1.000 |
| HFA227 | - | 9.868 | 14.802 | 19.736 |
| HFA 134a | 98.671 | 88.808 | 83.877 | 78.945 |

The formulations were compared to Example 11. Example 11 was found to provide the slowest sedimentation rate. Decreasing levels of HFA 227 resulted in more rapid sedimentation rates,

### Example 21

The formulation reported in the following Table was prepared in which all parts are by weight.

| | |
|---|---|
| Example | 21 |
| Budesonide | 0.280 |
| Ethanol | 1.000 |
| Oleic acid | 0.050 |
| HFA 227 | 98.670 |
| HFA 134a | - |

The formulation of Example 21 creamed.

## Claims

1. A pharmaceutical aerosol formulation in suspension form suitable for administration to a patient by oral or nasal inhalation consisting of:
particulate budesonide,
hydrofluoroalkane propellant selected from HFA 134a, HFA 227 and mixtures thereof,
from about 1 to 2% by weight of an adjuvant having a Kauri-butanol value of at least 10 and
optionally from 0.0001 to 1% by weight of the formulation of a surfactant selected from oleic acid, sorbitan oleates and lecithin.

2. A pharmaceutical aerosol formulation as claimed in Claim 1 in which the hydrofluoroalkane propellant is a mixture of HFA 134a and HFA 227 and in which the liquid mixture has a density at 20°C substantially equal to the density of the particulate budesonide.

3. A pharmaceutical aerosol formulation as claimed in Claim 1 or Claim 2 containing HFA 134a as a hydrofluoroalkane propellant.

4. A pharmaceutical aerosol formulation as claimed in Claim 1 or Claim 2 containing HFA 227 as a hydrofluoroalkane propellant.

5. A pharmaceutical aerosol formulation as claimed in any preceding Claim containing a propellant mixture of 15 to 35% by weight HFA 227 and from 65 to 85% by weight HFA 134a.

6. A pharmaceutical aerosol formulation as claimed in any preceding Claim in which the budesonide is present in an amount of from 1 to 8 mg/ml of formulation.

7. A pharmaceutical aerosol formulation as claimed in any preceding Claim containing from 0.0005 to 0.01 % by weight of surfactant.

8. A pharmaceutical aerosol formulation as claimed in any preceding claim In which the surfactant Is oleic acid.

9. A pharmaceutical aerosol formulation as claimed in any preceding Claim in which the adjuvant is ethanol.

10. A pharmaceutical aerosol formulation as claimed in Claim 9 comprising about 1% by weight of ethanol.

11. A pharmaceutical product comprising an aerosol canister equipped with a metered dose dispensing valve, the canister containing a pharmaceutical aerosol formulation as claimed in any preceding Claim.

## Patentansprüche

1. Pharmazeutische Aerosolformulierung in Suspensionsform, welche für die Verabreichung durch orale oder nasale Inhalation an einen Patienten geeignet ist, bestehend aus:
partikulärem Budesonid,
Hydrofluoralkan-Treibmittel, ausgewählt aus HFA 134a, HFA 227 und Gemischen davon,
etwa 1 bis 2 Gew.-% eines Adjuvans mit einem Kauri-Butanol-Wert von mindestens 10 und
gegebenenfalls 0,0001 bis 1 Gew.-% der Formulierung eines grenzflächenaktiven Stoffs, ausgewählt aus Ölsäure, Sorbitanoleaten und Lecithin.

2. Pharmazeutische Aerosolformulierung nach Anspruch 1, in welcher das Hydrofluoralkan-Treibmittel eine Mischung aus HFA 134a und HFA 227 ist und das Flüssiggemisch bei 20°C eine Dichte im wesentlichen gleich der Dichte des partikulären Budesonids aufweist.

3. Pharmazeutische Aerosolformulierung nach Anspruch 1 oder Anspruch 2, welche HFA 134a als ein Hydrofluoralkan-Treibmittel enthält.

4. Pharmazeutische Aerosolformulierung nach Anspruch 1 oder Anspruch 2, welche HFA 227 als ein Hydrofluoralkan-Treibmittel enthält.

5. Pharmazeutische Aerosolformulierung nach einem der vorstehenden Ansprüche, welche ein Treibmittelgemisch aus 15 bis 35 Gew.-% HFA 227 und 65 bis 85 Gew.-% HFA 134a enthält.

6. Pharmazeutische Aerosolformulierung nach einem der vorstehenden Ansprüche, in welcher das Budesonid in einer Menge von 1 bis 8 mg/ml der Formulierung vorliegt.

7. Pharmazeutische Aerosolformulierung nach einem der vorstehenden Ansprüche, welche 0,0005 bis 0,01 Gew.-% des grenzflächenaktiven Stoffs enthält.

8. Pharmazeutische Aerosolformulierung nach einem der vorstehenden Ansprüche, in welcher der grenzflächenaktive Stoff Ölsäure ist.

9. Pharmazeutische Aerosolformulierung nach einem der vorstehenden Ansprüche, in welcher das Adjuvans Ethanol ist.

10. Pharmazeutische Aerosolformulierung nach Anspruch 9, welche etwa 1 Gew.-% Ethanol umfasst.

11. Pharmazeutisches Produkt, umfassend einen Aerosolbehälter, welcher mit einem Ventil ausgestattet ist, durch das abgemessene Dosen abgegeben werden können, wobei der Behälter eine pharmazeutische Aerosolformulierung nach einem vorstehenden Ansprüche enthält.

## Revendications

1. Formulation pharmaceutique d'aérosol sous forme de suspension appropriée pour l'administration à un patient par inhalation orale ou nasale constituée :
de budésonide particulaire,
d'un propulseur hydrofluoroalcane choisi parmi HFA 134a, HFA 227 et leurs mélanges,
d'environ 1 à 2 % en poids d'un adjuvant ayant un indice de kauri-butanol d'au moins 10 et
éventuellement de 0,0001 à 1 % en poids de la formulation d'un agent tensioactif choisi parmi l'acide oléique, les oléates de sorbitane et la lécithine.

2. Formulation pharmaceutique d'aérosol selon la revendication 1 dans laquelle le propulseur hydrofluoroalcane est un mélange de HFA 134a et de HFA 227 et dans laquelle le mélange liquide a une masse volumique à 20 °C essentiellement égale à la masse volumique du budésonide particulaire.

3. Formulation pharmaceutique d'aérosol selon la revendication 1 ou la revendication 2 contenant HFA 134a comme propulseur hydrofluoroalcane.

4. Formulation pharmaceutique d'aérosol selon la revendication 1 ou la revendication 2 contenant HFA 227 comme propulseur hydrofluoroalcane.

5. Formulation pharmaceutique d'aérosol selon l'une quelconque des revendications précédentes contenant un mélange de propulseurs de 15 à 35 % en poids de HFA 227 et de 65 à 85 % en poids de HFA 134a.

6. Formulation pharmaceutique d'aérosol selon l'une quelconque des revendications précédentes dans laquelle le budésonide est présent en une quantité de 1 à 8 mg/ml de formulation.

7. Formulation pharmaceutique d'aérosol selon l'une quelconque des revendications précédentes contenant de 0,0005 à 0,01 % en poids d'agent tensio-actif.

8. Formulation pharmaceutique d'aérosol selon l'une quelconque des revendications précédentes dans laquelle l'agent tensioactif est l'acide oléique.

9. Formulation pharmaceutique d'aérosol selon l'une quelconque des revendications précédentes dans laquelle l'adjuvant est l'éthanol.

10. Formulation pharmaceutique d'aérosol selon la revendication 9 comprenant environ 1 % en poids d'éthanol.

11. Produit pharmaceutique comprenant une cartouche d'aérosol avec une vanne d'administration doseuse, la cartouche contenant une formulation pharmaceutique d'aérosol selon l'une quelconque des revendications précédentes.
